# EUROPEAN PATENT APPLICATION

(11) **EP 4 660 908 A1**
(43) Date of publication of application: **10.12.2025**
(21) Application number: 25179951.6
(22) Date of filing: 30.05.2025
(51) Int. Cl.: G06Q 10/06, B65B 9/08, B65B 37/18, B65B 57/00, B65B 57/14, B65B 61/02, B65B 65/00, B65B 65/02, G01G 19/393, G01N 1/00, G01N 1/28, G01N 37/00, G05B 19/418, G06Q 10/087, B65B 1/34

(54) **PRODUCTION MANAGEMENT APPARATUS**

(30) Priority: 03.06.2024 JP 2024090127
(71) Applicant: Ishida Co., Ltd., Kyoto-shi Kyoto 606-8392 (JP)
(72) Inventor: IWAGAKI, Keita, Ritto-shi, Shiga, 520-3026 (JP); KOIKE, Shinji, Ritto-shi, Shiga, 520-3026 (JP); YAMASHITA, Bunpei, Ritto-shi, Shiga, 520-3026 (JP); SHIBA, Kyohei, Ritto-shi, Shiga, 520-3026 (JP)
(74) Representative: Gill Jennings & Every LLP

(57) **Abstract**

A production management apparatus 4 manages a production line 100. The production management apparatus 4 includes a communication unit 4D configured to communicate with an information providing unit 31A configured to provide identification information to a packaging material F; and a control unit 4C configured to generate the identification information to be transmitted to the information providing unit 31A, and configured to transmit the generated identification information via the communication unit 4D. While the production line 100 produces a product M, when the control unit 4C executes intermediate inspection control to inspect whether inspection functions of inspection apparatuses 5, 6, and 7 operate normally, the control unit 4C transmits specific identification information, which is the identification information specifying that the product is a product for inspection, to the information providing unit 31A.

## Description

### TECHNICAL FIELD

One aspect of the present invention relates to a production management apparatus.

### BACKGROUND

As a production line for products such as confectionery, for example, a Patent Literature 1 (Japanese Unexamined Patent Publication No. 2023-135144) discloses a series of production lines including a combination weighing apparatus that weighs out objects to be weighed having variations in individual weight so as to achieve a target weight, and a packaging apparatus that manufactures bags from film and that stores the objects to be weighed, which are weighed out by the combination weighing apparatus, into the manufactured bags. In such a production line, for example, from the start of operation of the production line to the end of operation, it is periodically checked whether various inspection apparatuses that inspect whether products are produced according to predetermined specifications and whether there are any problems with the products function correctly (hereinafter, such checks will be referred to as an "intermediate inspection").

However, since such an intermediate inspection is performed via human intervention, work effort is required. In addition, since such an intermediate inspection involves inputting products for inspection, which lead to an abnormal inspection result when inspected from the inspection apparatuses, into the production line, and checking whether the products are correctly inspected by the inspection apparatuses, the production of the products is interrupted and the intermediate inspection is performed such that the products for inspection are not mixed as normal products.

### SUMMARY

Therefore, an object of one aspect of the present invention is to provide a production management apparatus capable of reducing the effort required to work and preventing products for inspection from being mixed in with normal products when an intermediate inspection is executed.
(1) A production management apparatus according to one aspect of the present invention is a production management apparatus that manages a production line, including: a communication unit that communicates with an information providing unit that provides identification information to a packaging material that stores articles; and a control unit that generates the identification information to be transmitted to the information providing unit, and that transmits the generated identification information via the communication unit. The control unit transmits specific identification information to the information providing unit while the production line produces a product in which the articles are stored by the packaging material when the control unit executes intermediate inspection control to inspect whether an inspection function of an inspection apparatus that inspects the product operates normally, the specific identification information being the identification information specifying that the product is a product for inspection.
   In the production management apparatus with this configuration, when an intermediate inspection is executed, the specific identification information specifying that the product is the product for inspection is generated, and the specific identification information is automatically provided to the product for inspection. Accordingly, even when the product for inspection is passed through the production line, there is no need to perform a process for enabling the product to be easily distinguished as the product for inspection via human intervention. In addition, since the specific identification information that enables the product to be easily distinguished as the product for inspection is provided to the product for inspection, the product for inspection can be easily removed after the intermediate inspection. As a result, when the intermediate inspection is executed, the effort required for work can be reduced and the product for inspection can be prevented from being mixed in with normal products.
(2) In the production management apparatus according to (1) above, the production line may include a weighing apparatus that weighs the articles, and a packing apparatus that stores the articles weighed by the weighing apparatus into the packaging material to form the product. When the control unit executes the intermediate inspection control, the control unit may transmit an instruction signal to the weighing apparatus, and cause the weighing apparatus, which has received the instruction signal, to supply the articles having weights outside a predetermined weight range to the packing apparatus. In this configuration, the product for inspection that becomes either an underweight product or an overweight product can be produced without human intervention. Accordingly, whether the inspection function of the weight inspection apparatus operates normally can be easily inspected without human effort.
(3) The production management apparatus according to [2] above may further include a foreign matter supply unit that supplies a pseudo foreign matter to the articles stored by the packing apparatus. When the control unit executes the intermediate inspection control, the control unit may transmit an instruction signal to the foreign matter supply unit, and cause the foreign matter supply unit, which has received the instruction signal, to supply the pseudo foreign matter to the packing apparatus. In this configuration, the product for inspection that contains the foreign matter can be produced without human intervention. Accordingly, whether the inspection function of an X-ray inspection apparatus or a metal detection apparatus operates normally can be easily inspected without human effort.
(4) The production management apparatus according to any one of [1] to [3] above may further include a notification unit that notifies that the intermediate inspection control is in execution. In this configuration, the product for inspection can be reliably prevented from being mixed in with normal products.
(5) The production management apparatus according to any one of [1] to [4] above may further include a pinhole forming unit that forms a pinhole in the packaging material to which the specific identification information is provided. When the control unit executes the intermediate inspection control, the control unit may transmit an instruction signal to the pinhole forming unit, and cause the pinhole forming unit, which has received the instruction signal, to form a pinhole in the packaging material. In this configuration, the product for inspection having an abnormality in the packaging material can be produced without human intervention. Accordingly, whether the inspection function of a seal inspection apparatus operates normally can be easily inspected without human effort.
(6) The production management apparatus according to any one of [1] to [5] above may further include a storage unit that stores an inspection result from the inspection apparatus and the specific identification information in association with each other. In this configuration, the inspection results of various inspection apparatuses for the product for inspection can be kept as records.

According to one aspect of the present invention, when the intermediate inspection is executed, the effort required for work can be reduced and the product for inspection can be prevented from being mixed in with normal products.

### BRIEF DESCRIPTION OF THE DRAWINGS

FIG. 1 is a schematic configuration of a production line managed by a production management apparatus according to one embodiment.
FIG. 2 is a view showing a schematic configuration of a combination weighing apparatus.
FIG. 3 is a view showing a schematic configuration of a bag-making and packaging apparatus.
FIG. 4 is a block diagram showing a functional configuration of the production management apparatus.
FIG. 5 is a block diagram showing a functional configuration of a production management apparatus according to a modification example.

### DETAILED DESCRIPTION

Hereinafter, a production management apparatus 4 according to one embodiment will be described with reference to the drawings. In the description of the drawings, the same elements are denoted by the same reference signs, and duplicate descriptions will be omitted.

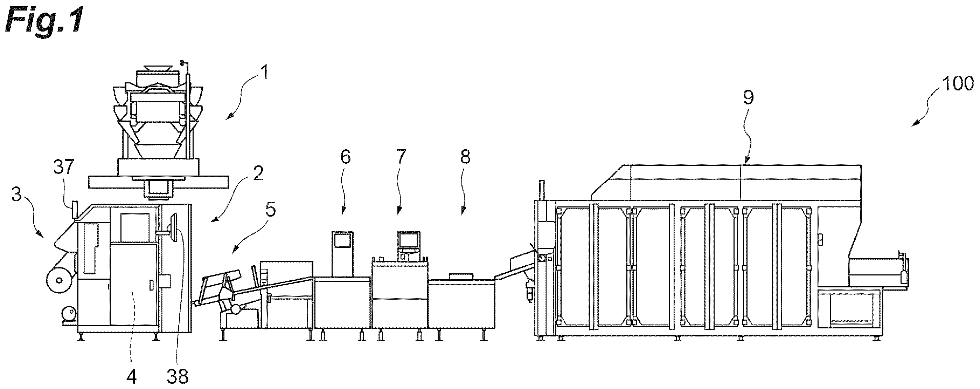

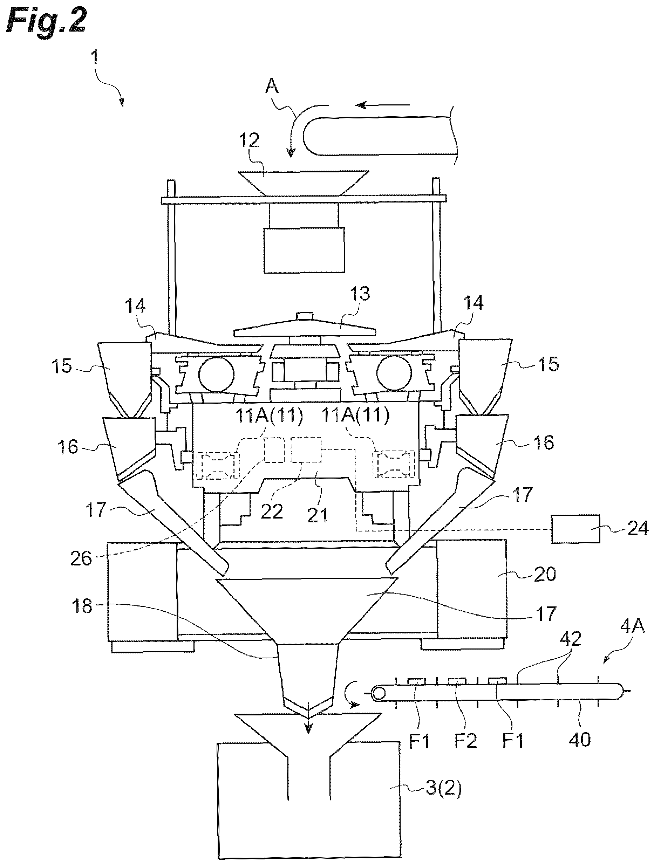

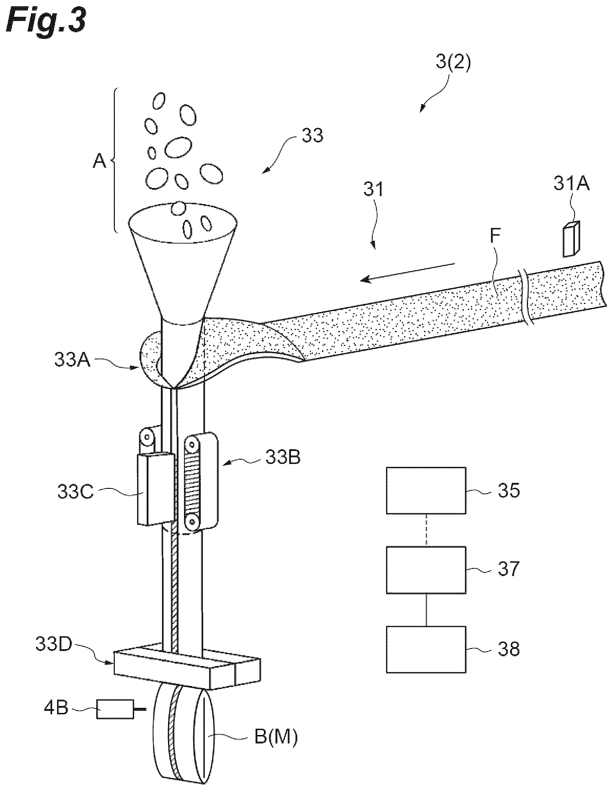

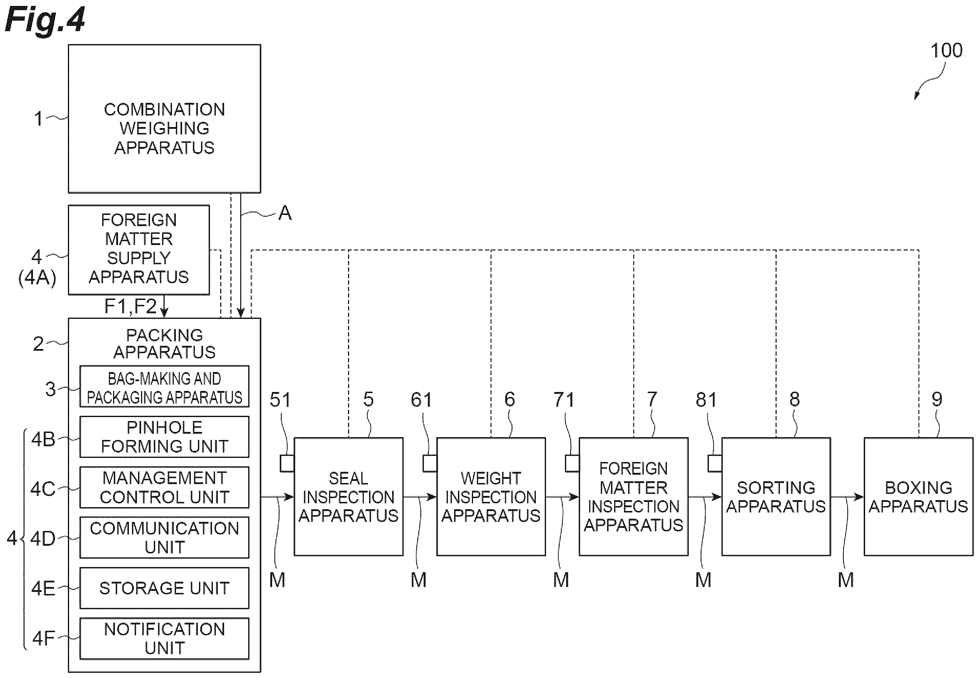

As shown in FIGS. 1 and 4, the production management apparatus 4 is a apparatus that manages a production line 100 including a combination weighing apparatus (weighing apparatus) 1 that weighs articles A (see FIGS. 2 and 3); a packing apparatus 2 for storing the articles A weighed by the combination weighing apparatus 1 in bags B (see FIG. 3), which are formed from a packaging film (packaging material) F, to form products M; a seal inspection apparatus 5, a weight inspection apparatus 6, and a foreign matter inspection apparatus 7 serving as inspection apparatuses that inspects the products M; a sorting apparatus 8 that discharges the products M, which are determined to be defective products by the inspection apparatuses, from the system; and a boxing apparatus 9 that boxes the products M.

First, the production line 100 managed by the production management apparatus 4 will be described. As shown in FIGS. 1 and 4, the production line 100 includes the combination weighing apparatus 1, the packing apparatus 2, the seal inspection apparatus 5, the weight inspection apparatus 6, the foreign matter inspection apparatus 7, the sorting apparatus 8, and the boxing apparatus 9. An apparatus group including the combination weighing apparatus 1, the bag-making and packaging apparatus 3, the seal inspection apparatus 5, the weight inspection apparatus 6, the sorting apparatus 8, and the boxing apparatus 9 constitute the production line 100 that executes a series of operations from weighing and packaging of the articles A to inspection of the products M, discharge of defective products from the system, and boxing. The articles A are, for example, articles having variations in individual mass, such as snacks, agricultural products, marine products, or processed foods.

The combination weighing apparatus 1 is an apparatus that performs combination weighing of the articles A. As shown in FIG. 2, the combination weighing apparatus 1 includes an input chute 12, a dispersion feeder 13, a plurality of radial feeders 14, a plurality of pool hoppers 15, a plurality of weighing hoppers 16, a collection chute 17, a timing hopper 18, a weighing unit 11, a display operation unit 24, and a weighing control unit 22. The combination weighing apparatus 1 repeats a cycle in which the articles A are supplied to the plurality of weighing hoppers 16 and are weighed, combinations of a plurality of the weighing hoppers 16 are obtained based on the obtained weighing value of each article in the plurality of weighing hoppers 16, a combination of the articles A, the weight value of which is closest to a target weight value, is selected from among the obtained combinations, and the articles are discharged from the plurality of weighing hoppers 16 related to the selected combination.

The weighing unit 11 is disposed inside a casing 21 supported by a frame 20. The weighing unit 11 includes a plurality of load cells 11A. Each of the load cells 11A supports the corresponding weighing hopper 16. When the article A is temporarily stored in each of the weighing hoppers 16, the weighing unit 11 weighs (acquires) the weight value of each weighing hopper (a weighing value corresponding to the mass of the article A).

The weighing control unit 22 is disposed inside the casing 21. The weighing control unit 22 includes a central processing unit (CPU), a read only memory (ROM), a random access memory (RAM), and the like. The weighing control unit 22 controls each part constituting the combination weighing apparatus 1. As shown in FIG. 2, the weighing control unit 22 is communicably connected to the production management apparatus 4 via a communication unit 4D.

The weighing control unit 22 stores a weighing value measured by the weighing unit 11 and the weighing hopper 16, which stores the article A corresponding to the weighing value, in association with each other. The weighing control unit 22 selects a combination of the articles A from a plurality of the weighing values of the articles A, which are weighed by the weighing unit 11 and are associated with the respective weighing hoppers 16, such that the total value becomes the target weight value. More specifically, the weighing control unit 22 combines the weighing values of the articles A output by the weighing unit 11, and selects a combination (calculates a combination) of the articles A such that the total combination value is close to the target weight value and falls within a predetermined range. Then, the weighing control unit 22 causes the articles A to be discharged from the weighing hoppers 16 for the articles A related to the calculated combination.

The packing apparatus 2 shown in FIGS. 1 and 3 includes the bag-making and packaging apparatus 3 and the production management apparatus 4. The bag-making and packaging apparatus 3 is an apparatus that manufactures the product M by storing the articles A, the combination of which is calculated by the combination weighing apparatus 1, in the bag B while making the bag B for storing the articles A from the packaging film F. The bag-making and packaging apparatus 3 bags the weighed articles A supplied from the combination weighing apparatus 1. As shown in FIG. 3, the bag-making and packaging apparatus 3 includes a film supply unit 31, a bag-making and packaging unit 33, and a packaging control unit 35.

The film supply unit 31 is a unit that supplies the packaging film F having a sheet shape to the bag-making and packaging unit 33. The film supply unit 31 includes a print unit (information providing unit) 31A that prints (provides) a two-dimensional code, in which at least bag serial number (identification information) is encoded, on the packaging film F. An example of the two-dimensional code is, for example, a QR code (registered trademark). The bag serial number is, for example, a number that can uniquely distinguish the bags B (products M) manufactured in a certain period of time. The print unit 31A is communicably connected to a management control unit (control unit) 4C to be described later in detail later.

The bag-making and packaging unit 33 is a unit that makes the bag B, in which the articles A is stored, by molding the packaging film F to form a tubular film that is a bag-shaped molded body for storing the articles A, and then cutting the packaging film F. The bag-making and packaging unit 33 includes a molding mechanism 33A that molds the packaging film F having a sheet shape into a tubular film; a pull-down belt mechanism 33B that conveys the packaging film molded in a tube shape (tubular film) downward; a vertical seal mechanism 33C that vertically seals an overlapping portion at both ends of the tubular film; and a horizontal seal mechanism 33D that horizontally seals the bag B formed from the tubular film, and that simultaneously heat-seals an upper end portion of the bag B and a lower end portion of the subsequent bag (tubular film).

The packaging control unit 35 is disposed inside a frame (not shown). The packaging control unit 35 includes a CPU, a ROM, a RAM, and the like. The packaging control unit 35 controls each part constituting the bag-making and packaging apparatus 3. As shown in FIG. 4, the bag-making and packaging apparatus 3 is communicably connected to the production management apparatus 4.

The seal inspection apparatus 5 shown in FIGS. 1 and 4 is an apparatus that inspects the bag thickness of the products M manufactured in the packing apparatus 2 and the presence or absence of air leakage. The seal inspection apparatus 5 includes a code reader 51. The code reader 51 reads a two-dimensional code printed on the bag B of the product M that is conveyed to the seal inspection apparatus 5. The seal inspection apparatus 5 is communicably connected to the production management apparatus 4 and the sorting apparatus 8 via the communication unit 4D. The seal inspection apparatus 5 transmits seal inspection information, which associates bag serial information included in the two-dimensional code read via the code reader 51 with the inspection result (bag thickness, presence or absence of leakage, and the like), to the production management apparatus 4.

The weight inspection apparatus 6 is an apparatus that inspects the weight of the product M manufactured in the packing apparatus 2. The weight inspection apparatus 6 includes a code reader 61. The code reader 61 reads a two-dimensional code printed on the bag B of the product M that is conveyed to the weight inspection apparatus 6. The weight inspection apparatus 6 is communicably connected to the production management apparatus 4 and the sorting apparatus 8 via the communication unit 4D. The weight inspection apparatus 6 transmits weight inspection information, which associates bag serial information included in the two-dimensional code read via the code reader 61 with the inspection result (proper weight, overweight, underweight, or the like), to the production management apparatus 4.

The foreign matter inspection apparatus 7 is an apparatus that inspects whether a foreign matter is mixed into the product M manufactured in the packing apparatus 2. Examples of the foreign matter inspection apparatus 7 include a metal detection apparatus, an X-ray inspection apparatus, and the like. The foreign matter inspection apparatus 7 includes a code reader 71. The code reader 71 reads a two-dimensional code printed on the bag B of the product M that is conveyed to the foreign matter inspection apparatus 7. The foreign matter inspection apparatus 7 is communicably connected to the production management apparatus 4 and the sorting apparatus 8 via the communication unit 4D. The foreign matter inspection apparatus 7 transmits foreign matter inspection information, which associates bag serial information included in the two-dimensional code read via the code reader 71 with the inspection result (presence or absence of a foreign matter and the like), to the production management apparatus 4.

The sorting apparatus 8 discharges the product M determined to be a defective product by the seal inspection apparatus 5, the weight inspection apparatus 6, or the foreign matter inspection apparatus 7 and the product M, on the bag B of which a two-dimensional code in which specific identification information specifying that the product is a product for inspection is encoded is printed, from the system, and the product M determined to be a non-defective product to a downstream step (boxing apparatus 9). Examples of the sorting apparatus 8 include an arm sorting apparatus using an arm, a drop-up belt sorting apparatus, a pusher sorting apparatus using a pusher apparatus, a drop flap sorting apparatus, an air jet sorting apparatus, a fin sorting apparatus, and the like. The sorting apparatus 8 includes a code reader 81. The code reader 81 reads a two-dimensional code printed on the bag B of the product M that is conveyed to the sorting apparatus 8. The sorting apparatus 8 is communicably connected to the production management apparatus 4, the seal inspection apparatus 5, the weight inspection apparatus 6, or the foreign matter inspection apparatus 7 via the communication unit 4D. The sorting apparatus 8 transmits sorting information, which associates bag serial information included in the two-dimensional code read via the code reader 81 with the sorting result (presence or absence of discharge of the products M from the system), to the production management apparatus 4.

The boxing apparatus 9 is an apparatus that packs the products M into cardboard boxes. The boxing apparatus 9 packs a plurality of the products M into a cardboard box, and conveys the cardboard box to a downstream step.

The production management apparatus 4 includes a foreign matter supply apparatus (foreign matter supply unit) 4A, a pinhole forming unit 4B, the management control unit 4C, the communication unit 4D, a storage unit 4E, and a notification unit 4F. The pinhole forming unit 4B, the management control unit 4C, the communication unit 4D, the storage unit 4E, and the notification unit 4F are provided in the packing apparatus 2.

The foreign matter supply apparatus 4A supplies a pseudo foreign matter F1 (F2) to the articles A that are stored in the packaging film F in the packing apparatus 2. The pseudo foreign matter F1 (F2) is, for example, the pseudo foreign matter F1 (F2) such as metal that should be detected by the foreign matter inspection apparatus 7. As shown in FIG. 2, the foreign matter supply apparatus 4A is disposed between the combination weighing apparatus 1 and the packing apparatus 2. The foreign matter supply apparatus 4A is composed of a belt conveyor. A belt 40 used in the foreign matter supply apparatus 4A is provided with partitioning portions 42 that partition the belt 40 at predetermined spacings in a conveying direction, and one pseudo foreign matter F1 (F2) is disposed at each disposition location partitioned by the partitioning portions 42. In the present embodiment, the pseudo foreign matters F1 (F2) of two types having different physical properties are disposed at the respective disposition locations. The product M for inspection in which the pseudo foreign matter F1 is stored and the product M for inspection in which the pseudo foreign matter F2 is stored are manufactured by supplying the pseudo foreign matters F1 (F2) from the foreign matter supply apparatus 4A. The operation of the foreign matter supply apparatus 4A (driving of the belt conveyor) is controlled by the management control unit 4C.

The pinhole forming unit 4B forms a pinhole in the bag B (product M) in which the articles A are stored and which is manufactured in the bag-making and packaging apparatus 3 of the packing apparatus 2. As shown in FIG. 3, the pinhole forming unit 4B is disposed, for example, adjacent to the downstream side of the horizontal seal mechanism 33D. The pinhole forming unit 4B includes a needle and a drive unit that moves the needle toward and away from the bag B. The pinhole forming unit 4B forms a pinhole in the bag B by approaching the bag B and piercing the bag B with the needle. The operation of the pinhole forming unit 4B (driving of the drive unit) is controlled by the management control unit 4C.

The management control unit 4C shown in FIGS. 1 and 4 controls each part of the production management apparatus 4 and executes various functions in the production management apparatus 4. The management control unit 4C includes a CPU, a ROM, a RAM, and the like. The management control unit 4C can be configured, for example, as software in which a program stored in the ROM is loaded onto the RAM and is executed by the CPU. The management control unit 4C may be configured as hardware such as an electronic circuit.

The management control unit 4C controls the print unit 31A to print a two-dimensional code, in which a bag serial number (identification information) is encoded, on the packaging film F of the articles A (the packaging film F forming the bag B in which the articles A are stored). In more detail, the management control unit 4C issues (generates) a dedicated bag serial number for each bag B that is formed, and controls the print unit 31A to print a two-dimensional code, in which the issued bag serial number is encoded, on the packaging film F. The print unit 31A that has received the bag serial number prints the bag serial number on the packaging film F forming the bag B into which the articles A discharged from the combination weighing apparatus 1 are packaged.

The management control unit 4C executes intermediate inspection control to inspect whether the inspection function of the inspection apparatus operates normally while the production line 100 produces the products M. For example, the execution of the intermediate inspection control may be automatically started based on a setting determined in the storage unit 4E or the like in advance, or may be started in response to a trigger such as the pressing of a start button displayed on a display operation unit 38 by a worker or the like on the production line 100. Examples of the setting determined in advance include a setting where the intermediate inspection control is started when a predetermined number (for example, 1000) of the products M are manufactured, a setting where the intermediate inspection control is started when a predetermined time (for example, one hour) has elapsed, a setting where the intermediate inspection control is started on a predetermined schedule (set time), and the like.

When the management control unit 4C of the present embodiment executes the intermediate inspection control, the management control unit 4C generates specific identification information that is identification information specifying that the product is a product for inspection, and transmits the specific identification information to the print unit 31A via the communication unit 4D. The print unit 31A prints the specific identification information on the bag B into which the articles A discharged from the combination weighing apparatus 1 are packaged.

When the management control unit 4C executes the intermediate inspection control, the management control unit 4C transmits an instruction signal to the combination weighing apparatus 1 and causes the combination weighing apparatus 1, which has received the instruction signal, to supply the articles A having weights outside a predetermined weight range to the packing apparatus 2 (hereinafter, also referred to as "weight inspection control"), transmits an instruction signal to the foreign matter supply apparatus 4A and causes the foreign matter supply apparatus 4A, which has received the instruction signal, to supply the pseudo foreign matters F1 (F2) to the packing apparatus 2 (hereinafter, also referred to as "foreign matter inspection control"), or transmits an instruction signal to the pinhole forming unit 4B and causes the pinhole forming unit 4B, which has received the instruction signal, to form a pinhole in the bags B (hereinafter, also referred to as "pinhole inspection control").

Here, the operation of the bag-making and packaging apparatus 3 for manufacturing the product M for inspection when the management control unit 4C executes the foreign matter inspection control will be described. When the production management apparatus 4 executes the foreign matter inspection control, the production management apparatus 4 acquires, via the communication unit 4D, a verification signal for manufacturing the product M for inspection to inspect the inspection function of the inspection apparatus disposed downstream of the packing apparatus 2 during the manufacture of the products M. Examples of the verification signal include a press signal that is received when a worker presses the start button displayed on the display operation unit 38 of the bag-making and packaging apparatus 3, a start signal that is transmitted based on a schedule for starting an intermediate inspection, which is set in advance, and the like.

Upon receiving the verification signal, the management control unit 4C transmits an input signal for inputting the pseudo foreign matter F1 (F2) into the bag B. In addition, upon acquiring the verification signal, the management control unit 4C causes the print unit 31A to print a two-dimensional code, in which specific identification information is encoded, on the packaging film F, continues the manufacture of the products M until the packaging film F on which the two-dimensional code is printed reaches the bag-making and packaging unit 33, and transmits an input signal at the timing the packaging film F on which the specific identification information is encoded reaches the bag-making and packaging unit 33. The management control unit 4C may temporarily interrupt the conveyance of the packaging film F at the timing the verification signal is acquired, namely, when the content is switched from the content printed when the normal products M are manufactured to the content printed on the products M for inspection.

When the packaging film F on which the specific identification information is encoded is conveyed to the bag-making and packaging unit 33, the management control unit 4C temporarily interrupts the conveyance of the packaging film F before the pseudo foreign matter F1 (F2) is input into the bag B formed from the packaging film F. At this timing, the management control unit 4C may check whether the two-dimensional code that has reached the bag-making and packaging unit 33 is the two-dimensional code in which the specific identification information is encoded. In addition, even when the packaging film F on which the specific identification information is encoded is conveyed to the bag-making and packaging unit 33, the management control unit 4C may cause the packaging film F to be conveyed without temporarily interrupting the conveyance of the packaging film F. When the input of the pseudo foreign matter F1 (F2) into the bag B formed from the packaging film F is completed, the management control unit 4C causes the input of the articles A to the bag B formed from the packaging film F to restart in the bag-making and packaging unit 33.

In addition, here, the operation of the bag-making and packaging apparatus 3 for manufacturing the product M for inspection when the management control unit 4C executes the weight inspection control will be described. Similarly to when the management control unit 4C executes the foreign matter inspection control, the management control unit 4C also acquires a verification signal via the communication unit 4D when the management control unit 4C executes the weight inspection control. Upon receiving the verification signal, the management control unit 4C transmits an input signal for inputting the articles A, the inspection weighing values of which have a condition different from that of the set range, into the bag B to the combination weighing apparatus 1. In addition, upon acquiring the verification signal, the management control unit 4C causes the print unit 31A to print a two-dimensional code, in which specific identification information is encoded, on the packaging film F, continues the manufacture of the products M until the packaging film F on which the two-dimensional code is printed reaches the bag-making and packaging unit 33, and transmits an input signal at the timing the packaging film F on which the two-dimensional code in which the specific identification information is encoded is printed reaches the bag-making and packaging unit 33. Conveyance control of the packaging film F by the management control unit 4C is also performed in the similar manner as the foreign matter inspection control.

In addition, here, the operation of the bag-making and packaging apparatus 3 for manufacturing the product M for inspection when the management control unit 4C executes the pinhole inspection control will be described. Similarly to when the management control unit 4C executes the foreign matter inspection control, the management control unit 4C also acquires a verification signal via the communication unit 4D when the management control unit 4C executes the pinhole inspection control. Upon receiving the verification signal, the management control unit 4C transmits a formation signal for forming a pinhole in the bag B to the pinhole forming unit 4B. In addition, upon acquiring the verification signal, the management control unit 4C causes the print unit 31A to print a two-dimensional code, in which specific identification information is encoded, on the packaging film F, continues the manufacture of the products M until the packaging film F on which the two-dimensional code is printed reaches the pinhole forming unit 4B of the bag-making and packaging unit 33, and transmits an input signal at the timing the packaging film F on which the two-dimensional code in which the specific identification information is encoded is printed reaches the pinhole forming unit 4B of the bag-making and packaging unit 33. Conveyance control of the packaging film F by the management control unit 4C is also performed in the similar manner as the foreign matter inspection control.

In various inspection controls (the foreign matter inspection control, the weight inspection control, or the pinhole inspection control), the management control unit 4C automatically stops the operation of the production line 100 when the manufacture of a predetermined number of the products M for inspection is completed and the completion of the manufacture of the products M for inspection is not detected within a predetermined time, and automatically switches to the manufacture of the normal products M when the completion of the manufacture of the products M for inspection is detected within the predetermined time (first control). The management control unit 4C may automatically switch to the manufacture of the normal products M (return to manufacture) at the timing the manufacture of the predetermined number of products M for inspection is completed, automatically stop the manufacture of the normal products M when the completion of the manufacture of the products M for inspection is not detected within the predetermined time, and continue the manufacture of the normal products M when the completion of the manufacture of the products M for inspection is detected within the predetermined time (second control). Whether the first control or the second control is performed in the management control unit 4C can be arbitrarily set. The completion of the manufacture of the products M for inspection can be detected, for example, upon the reading of a predetermined number of two-dimensional codes, in which specific identification information is encoded, by the code reader 81 of the sorting apparatus 8 or upon the inspection of a predetermined number of the products M for inspection by the foreign matter inspection apparatus 7.

In the management control unit 4C, which inspection apparatus is to be specified as the target of inspection when the intermediate inspection control is executed can be set in advance. For example, in a single execution of the intermediate inspection control, the management control unit 4C can be arbitrarily set to execute three controls: the weight inspection control for executing the inspection of the weight inspection apparatus 6, the foreign matter inspection control for executing the inspection of the foreign matter inspection apparatus 7, and the pinhole inspection control for executing the inspection of the seal inspection apparatus 5, or to execute only the weight inspection control for executing the inspection of the weight inspection apparatus 6. In addition, the management control unit 4C may change the inspection apparatus that is the target of inspection, each time the intermediate inspection control is executed.

The management control unit 4C stores the seal inspection information, which associates the bag serial information included in the two-dimensional code read via the code reader 51 with the inspection result (bag thickness, presence or absence of leakage, and the like) and which is transmitted from the seal inspection apparatus 5, in the storage unit 4E. The management control unit 4C stores the weight inspection information, which associates the bag serial information included in the two-dimensional code read via the code reader 61 with the inspection result (proper weight, overweight, underweight, or the like) and which is transmitted from the weight inspection apparatus 6, in the storage unit 4E. The management control unit 4C stores the foreign matter inspection information, which associates the bag serial information included in the two-dimensional code read via the code reader 71 with the inspection result (presence or absence of a foreign matter and the like) and which is transmitted from the foreign matter inspection apparatus 7, in the storage unit 4E. The management control unit 4C stores the sorting information, which associates the bag serial information included in the two-dimensional code read via the code reader 81 with the inspection result (presence or absence of discharge of the products M from the system) and which is transmitted from the sorting apparatus 8, in the storage unit 4E.

The communication unit 4D is an interface that enables communication with other apparatuses. The communication unit 4D is provided to be able to communicate with the combination weighing apparatus 1, the foreign matter supply apparatus 4A, the seal inspection apparatus 5, the weight inspection apparatus 6, the foreign matter inspection apparatus 7, the sorting apparatus 8, and the boxing apparatus 9. The communication between the apparatuses may be performed by wire or wirelessly.

The storage unit 4E is an apparatus that stores various information that is managed in the production management apparatus 4. The storage unit 4E is an apparatus such as a hard disk drive or a solid state drive. The storage unit 4E stores information (setting information) serving as a condition for starting the intermediate inspection control. In addition, the storage unit 4E stores the inspection records described above, namely, the seal inspection information, the weight inspection information, the foreign matter inspection information, and the sorting information.

The notification unit 4F notifies a worker or the like that the intermediate inspection control is in execution. Examples of the notification unit 4F include a signal tower 37 provided in the bag-making and packaging apparatus 3, the display operation unit 38, and the like. In addition, the notification unit 4F may be a signal tower or a display operation unit (for example, the display operation unit 24 of the combination weighing apparatus) mounted in various apparatuses other than the bag-making and packaging apparatus 3. In addition, the notification unit 4F may be a smartphone, a tablet, or the like that can be connected to the production management apparatus 4.

Actions and effects of the production management apparatus 4 of the above-described embodiment will be described. In the production management apparatus 4 of the above-described embodiment, when an intermediate inspection is executed, the specific identification information specifying that the product M is the product M for inspection is generated, and a two-dimensional code including the specific identification information is automatically printed on the bag B of the product M for inspection. Accordingly, even when the product M for inspection is passed through the production line 100, there is no need to perform a process for enabling the product M to be easily distinguished as the product M for inspection via human intervention. In addition, since a two-dimensional code including the specific identification information that enables the product M to be easily distinguished as the product M for inspection is printed on the product M for inspection, the product M for inspection can be easily removed after the intermediate inspection. As a result, when the intermediate inspection is executed, the effort required for work can be reduced and the product M for inspection can be prevented from being mixed in with normal products.

In the production management apparatus 4 of the above-described embodiment, when the management control unit 4C executes the intermediate inspection control, the management control unit 4C can transmit an instruction signal to the combination weighing apparatus 1 via the communication unit 4D, and cause the combination weighing apparatus 1, which has received the instruction signal, to supply the articles A having weights outside the predetermined weight range to the packing apparatus 2. Accordingly, the product M for inspection that becomes either an underweight product or an overweight product can be produced without human intervention. Accordingly, whether the inspection function of the weight inspection apparatus 6 serving as the inspection apparatus operates normally can be easily inspected without human effort.

In the production management apparatus 4 of the above-described embodiment, when the management control unit 4C executes the intermediate inspection control, the management control unit 4C can transmit an instruction signal to the foreign matter supply apparatus 4A, and cause the foreign matter supply apparatus 4A, which has received the instruction signal, to supply the pseudo foreign matter F1 (F2) to the packing apparatus 2. Accordingly, the product M for inspection that contains the pseudo foreign matter F1 (F2) can be produced without human intervention. Accordingly, whether the inspection function of the foreign matter inspection apparatus 7 serving as the inspection apparatus operates normally can be easily inspected without human effort.

Since the production management apparatus 4 of the above-described embodiment includes the notification unit 4F that notifies that the intermediate inspection control is in execution, a worker or the like can be made aware that the intermediate inspection is in execution. Accordingly, the product M for inspection can be more reliably prevented from being mixed in with normal products.

In the production management apparatus 4 of the above-described embodiment, when the management control unit 4C executes the intermediate inspection control, the management control unit 4C can transmit an instruction signal to the pinhole forming unit 4B, and cause the pinhole forming unit 4B, which has received the instruction signal, to form a pinhole in the bag B formed from the packaging film F. In this configuration, the product M for inspection having an abnormality in the packaging film F can be produced without human intervention. Accordingly, whether the inspection function of the seal inspection apparatus 5 serving as the inspection apparatus operates normally can be easily inspected without human effort.

Since the production management apparatus 4 of the above-described embodiment includes the storage unit 4E that stores the inspection results from the inspection apparatuses (the seal inspection apparatus 5, the weight inspection apparatus 6, and the foreign matter inspection apparatus 7) and the specific identification information in associated with each other, the inspection results of various inspection apparatuses for the product M for inspection can be kept as records.

One embodiment has been described above; however, one aspect of the present invention is not limited to the above-described embodiment. Various modifications can be made without departing from the concept of the invention.

In the above-described embodiment, the print unit 31A has been described as an example of the information providing unit that provides a two-dimensional code to the packaging film F; however, for example, a configuration in which a two-dimensional code is printed on a seal and the seal on which the two-dimensional code is printed is affixed may be adopted. In addition, in the above-described embodiment, an example in which a two-dimensional code is provided has been described; however, an identification number or a specific identification number may be printed as is. The identification number or the specific identification number can be composed of, for example, as a combination of numbers and letters; however, for example, any configuration such as numbers only, letters only, or a combination of these with symbols can be adopted as long as the configuration enables the product M to be distinguished from other products M.

In the above-described embodiment, an example in which an identification number or a specific identification number is printed on the packaging film F has been described; however, the present invention is not limited thereto. For example, an IC tag in which an identification number or a specific identification number is stored may be attached, or another code (one-dimensional code, symbol, pattern, or coloration) that can be uniquely converted into an identification number or a specific identification number may be used.

In the above-described embodiment and the above-described modification examples, an example in which a two-dimensional code is printed on the packaging film F forming the bag B in which the articles A are stored has been described; however, an identification number or a specific identification number may be printed on (provided to) the bag B (namely, after the bag B is formed) in which the articles A are stored.

In the above-described embodiment and the above-described modification examples, the bag-making and packaging apparatus 3 that stores the articles A in the formed bag B while forming the bag B for storing the articles A using the packaging film F has been described as an example of the packing apparatus 2; however, the present invention is not limited thereto. For example, the packing apparatus 2 may be configured to store the articles A in the bag B that is formed in advance, or may be configured to store the articles A in a ready-made box, container, or the like.

In the above-described embodiment and the above-described modification examples, the combination weighing apparatus 1 has been described as an example of the weighing apparatus; however, the present invention is not limited thereto, and the weighing apparatus may be a weighing apparatus that discharges the articles A having a fixed weight.

In the above-described embodiment and the above-described modification examples, the production line 100 composed of the apparatus group including the combination weighing apparatus 1, the packing apparatus 2, the seal inspection apparatus 5, the weight inspection apparatus 6, the foreign matter inspection apparatus 7, the sorting apparatus 8, and the boxing apparatus 9 has been described as an example; however, as long as the combination weighing apparatus 1 and the packing apparatus 2 are included, the seal inspection apparatus 5, the weight inspection apparatus 6, the foreign matter inspection apparatus 7, the sorting apparatus 8, and the boxing apparatus 9 may not necessarily all be included, only some of the apparatuses may be included, or other apparatuses may be included.

In the production management apparatus 4 of the above-described embodiment and the above-described modification examples, a configuration in which one foreign matter supply apparatus 4A that supplies the pseudo foreign matters F1 (F2) of two types is disposed has been described; however, the present invention is not limited thereto. For example, a configuration in which two foreign matter supply apparatuses 4A and 4A that supplies the pseudo foreign matter F1 and the pseudo foreign matter F2, respectively, may be adopted.

In the production management apparatus 4 of the above-described embodiment and the above-described modification examples, an example in which the foreign matter supply apparatus 4A and the pinhole forming unit 4B are included has been described; however, the two apparatuses may not be included or only one of the two apparatuses may be included. In addition, the pinhole forming unit 4B is not configured as a part of the bag-making and packaging apparatus 3, but may be incorporated into the production line 100 in a state where the pinhole forming unit 4B is independently configured.

In the production management apparatus 4 of the above-described embodiment and the above-described modification examples, an example in which the combination weighing apparatus 1 is controlled to form the product M for inspection in which the articles A stored in the bag B are excessive or insufficient has been described; however, the present invention is not limited thereto. For example, the production management apparatus 4 may manufacture the product M for inspection, in which the article A is jammed in a seal portion, by controlling the bag-making and packaging apparatus 3 to adjust the conveying speed of the tubular film, the horizontal seal timing, or the like. By manufacturing such a product M for inspection, whether the jamming inspection of an X-ray inspection apparatuses serving as the inspection apparatus functions correctly can be automatically inspected.

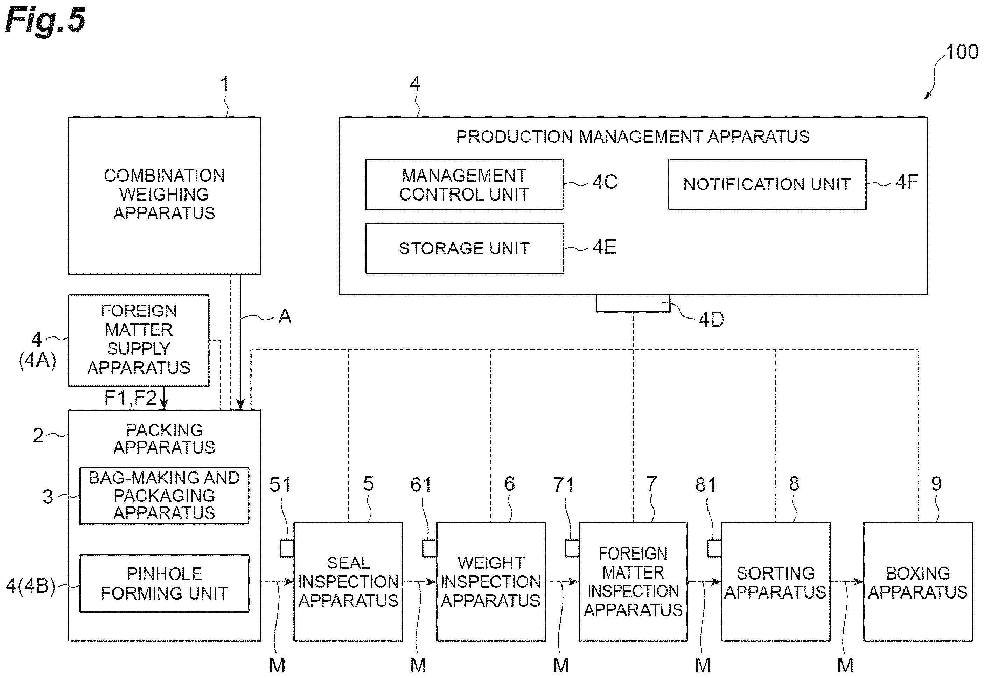

As described in FIG. 4, an example in which the production management apparatus 4 of the above-described embodiment and the above-described modification examples is formed integrally with the bag-making and packaging apparatus 3 serving as the packing apparatus 2 has been described; however, the production management apparatus 4 may be configured separately from the bag-making and packaging apparatus 3. For example, as shown in FIG. 5, the production management apparatus 4 may be configured to be able to communicate with the combination weighing apparatus 1, the bag-making and packaging apparatus 3 (packing apparatus 2), the seal inspection apparatus 5, the weight inspection apparatus 6, the foreign matter inspection apparatus 7, the sorting apparatus 8, and the boxing apparatus 9 via a network.

## Claims

1. A production management apparatus configured to manage a production line, comprising:
a communication unit configured to communicate with an information providing unit configured to provide identification information to a packaging material configured to stores articles; and
a control unit configured to generate the identification information to be transmitted to the information providing unit and transmits the generated identification information via the communication unit,
wherein the control unit transmits specific identification information to the information providing unit while the production line produces a product in which the articles are stored by the packaging material when the control unit executes intermediate inspection control to inspect whether an inspection function of an inspection apparatus that inspects the product operates normally, the specific identification information being the identification information specifying that the product is a product for inspection.

2. The production management apparatus according to claim 1,
wherein the production line includes a weighing apparatus configured to weigh the articles and a packing apparatus configured to store the articles weighed by the weighing apparatus in the packaging material to form the product, and
the control unit transmits an instruction signal to the weighing apparatus when executing the intermediate inspection control and causes the weighing apparatus receiving the instruction signal to supply the articles having weights outside a predetermined weight range to the packing apparatus.

3. The production management apparatus according to claim 2, further comprising:
foreign matter supply unit configured to supply pseudo foreign matter to the articles stored by the packing apparatus,
wherein the control unit transmits an instruction signal to the foreign matter supply unit when executing the intermediate inspection control and causes the foreign matter supply unit receiving the instruction signal to supply the pseudo foreign matter to the packing apparatus.

4. The production management apparatus according to any one of claims 1 to 3, further comprising:
a notification unit configured to notify that the intermediate inspection control is being executed.

5. The production management apparatus according to any one of claims 1 to 4, further comprising:
a pinhole forming unit configured to form a pinhole in the material to which the specific identification information is provided,
wherein the control unit transmits an instruction signal to the pinhole forming unit when executing the intermediate inspection control and causes the pinhole forming unit receiving the instruction signal to form a pinhole in the packaging material.

6. The production management apparatus according to any one of claims 1 to 5, further comprising:
a storage unit configured to store an inspection result from the inspection apparatus and the specific identification information in association with each other.
